# EUROPEAN PATENT APPLICATION

(11) **EP 4 596 004 A1**
(43) Date of publication of application: **06.08.2025**
(21) Application number: 24305171.1
(22) Date of filing: 31.01.2024
(51) Int. Cl.: A61M 5/32

(54) **NEEDLE COVER FOR MEDICAL INJECTION DEVICE**

(71) Applicant: Becton Dickinson France, 38800 Le Pont-de-Claix (FR)
(72) Inventor: NICOLAS, Maxime, 34570 Montarnaud (FR); VAXELAIRE, Jérémie, 38800 Le Pont de Claix (FR); DORELON, Luc, 38650 Saint Martin de la Cluz (FR); OZTURK, Senturk, 38130 Echirolles (FR); REMPFER, Simon, 38260 St Hilaire de la Cote (FR)
(74) Representative: Germain Maureau

(57) **Abstract**

A needle cover for protecting a needle mounted on a tip of a medical injection device, wherein the tip extends from a distal end of the medical injection device, including an inner shield extending along a longitudinal axis, and an outer shield surrounding at least partially the inner shield, and operatively connected to said inner shield. The outer shield may further include an extension strip provided on a proximal portion of the outer shield and extends in a distal direction to a position held between the inner shield and the tip.

## Description

### BACKGROUND OF THE INVENTION

### Field of the Invention

The present disclosure relates generally to medical injection devices for delivery of a fluid or liquid medicament. More particularly, the present disclosure relates to a needle cover adapted to be mounted on a tip of a medical injection device for covering a needle attached thereon. The disclosure also relates to a medical assembly including a medical injection device and a needle cover for enclosing the needle of the medical injection device.

### Description of Related Art

Medical injection devices such as syringes are well known in the art. These devices typically include a container for containing a medical composition such as a liquid medicament. Said container usually includes an end piece in a form of a longitudinal tip defining a fluid path through which the medical solution is expelled. A needle may be attached to the tip in order to prick the patient's skin and to perform the injection of the medical composition.

In order to maintain sterility prior to use and to reduce the risk of incurring an accidental needle-stick, protection of the needle is important. Thus, a needle cover may be mounted on the tip of the barrel so as to enclose the needle. This renders the needle physically inaccessible by the persons around the device. The needle cover may include an inner shield, in a material with elastomeric properties, and may further include an outer shield, in rigid plastic, surrounding the inner shield. In some examples, the inner needle shield may be made of a rubber material. The inner needle shield ensures the sealing of the medical injection device. To that purpose, the inner needle shield includes a sealing portion that sealingly contacts the outer surface of the syringe's tip to provide a tight seal. The inner needle shield prevents any contamination of the medical composition from the outside environment, thereby assuring the container closure integrity. The inner needle shield further prevents any leakage of composition from the outlet of the needle to the external environment. To that purpose, the needle is preferably pricked in the inner needle shield. The rigid outer shield may be made of a rigid or semi-rigid material that surrounds the inner needle shield.

The inner needle shield creates an interference force with the tip of the medical injection device to ensure a full closure of the liquid within the medical injection device. In some examples, the closure integrity of the opening of the medical injection device is created by a radial compression of the inner needle shield on the tip of the medical injection device. The radial compression on the tip of the medical injection device is increased below the tip of the medical injection device using the rigid outer needle shield to avoid any unintentional dislodgement of the needle cover off of the medical injection device. Many of these features of the needle cover also affect the amount of force needed to remove the needle cover from the medical injection device.

When designing a needle cover there are several features to take into consideration that affect the performance level of the needle cover. In particular, among these features are container closure integrity at the tip of the medical injection device, the unintentional dislodgement of the needle cover from the medical injection device, and the ability to pull the needle cover off of the medical injection device using a reasonable amount of force. These features of the needle cover often determine three different characteristics of the needle cover, which often makes it difficult to adjust a performance level for an individual feature without affecting the performance level of the remaining features.

### SUMMARY OF THE INVENTION

In view of the problems identified above, there is a current need for a needle cover that improves the container closure integrity at the tip of the medical injection device, reduces the possibility of an unintentional dislodgement of the needle cover from the medical injection device, and reduces the amount of force needed to remove the needle cover from the medical injection device.

According to one non-limiting embodiment or aspect, a needle cover for protecting a needle mounted on a tip of a medical injection device, wherein the tip extends from a distal end of the medical injection device, the needle cover, may include an inner shield extending along a longitudinal axis, and an outer shield surrounding at least partially the inner shield, and operatively connected to said inner shield. The outer shield may further include an extension strip provided on a proximal portion of the outer shield and extends in a distal direction to a position held between the inner shield and the tip.

According to one non-limiting embodiment or aspect, the extension strip may contact an inner surface of the inner shield when the outer shield is covering the needle. The extension strip may not contact the tip or the inner shield when the outer shield is covering the needle. The extension strip may be made of a same material as the outer shield. The extension strip may be made of a rigid plastic material. The extension strip may be formed integral with the outer shield. The outer shield may define at least one opening through which a proximal portion of the inner shield extends when the needle cover is covering the needle. As the outer shield is pulled or pushed in a distal direction, the proximal portion of the outer shield may contact the proximal portion of the inner shield held in the at least one opening of the outer shield to cause the inner shield to also move in the distal direction. As the inner shield is moved in the distal direction, the extension strip of the outer shield may interleave between the tip and the inner shield to peel or separate the inner shield from the tip. A longitudinal length of the at least one opening may be the same as or substantially similar to a longitudinal length of the extension strip of the outer shield.

According to one non-limiting embodiment or aspect, a medical injection device, may include a barrel extending a proximal face to a distal face, wherein a tip is provided on the distal face of the barrel, and a needle cover for protecting a needle mounted on the tip of the medical injection device, the needle cover may include an inner shield extending along a longitudinal axis, and an outer shield surrounding at least partially the inner shield, and operatively connected to said inner shield. The outer shield may include an extension strip provided on a proximal portion of the outer shield and extends in a distal direction to a position held between the inner shield and the tip.

According to one non-limiting embodiment or aspect, the extension strip may contact an inner surface of the inner shield when the outer shield is covering the needle. The extension strip may not contact the tip or the inner shield when the outer shield is covering the needle. The extension strip may be made of a same material as the outer shield. The extension strip may be made of a rigid plastic material. The extension strip may be formed integral with the outer shield. The outer shield may define at least one opening through which a proximal portion of the inner shield extends when the needle cover is covering the needle. As the outer shield is pulled or pushed in a distal direction, the proximal portion of the outer shield may contact the proximal portion of the inner shield held in the at least one opening of the outer shield to cause the inner shield to also move in the distal direction. As the inner shield is moved in the distal direction, the extension strip of the outer shield may interleave between the tip and the inner shield to peel or separate the inner shield from the tip. A longitudinal length of the at least one opening may be the same as or substantially similar to a longitudinal length of the extension strip of the outer shield. The inner shield may be made of a soft material including an elastomer or a thermoplastic elastomer.

The invention is further disclosed in the following clauses:
Clause 1: A needle cover for protecting a tip and a needle mounted on the tip of a medical injection device, wherein the tip extends from a distal end of the medical injection device, the needle cover, comprising: an inner shield extending along a longitudinal axis, and an outer shield surrounding at least partially the inner shield, and operatively connected to said inner shield, the needle cover being characterized in that: the outer shield further comprises an extension strip provided on a proximal portion of the outer shield and extends in a distal direction to a position held between the inner shield and the tip.
Clause 2: The needle cover of Clause 1, wherein the extension strip contacts an inner surface of the inner shield when the outer shield of the needle cover is covering the needle.
Clause 3: The needle cover of Clause 1, wherein the extension strip does not contact the tip or the inner shield when the outer shield of the needle cover is covering the needle.
Clause 4: The needle cover of Clause 1, wherein the extension strip is made of a same material as the outer shield.
Clause 5: The needle cover of Clause 1, wherein the extension strip is made of a rigid plastic material.
Clause 6: The needle cover of Clause 1, wherein the extension strip is formed integral with the outer shield.
Clause 7: The needle cover of Clause 1, wherein the outer shield defines at least one opening through which a proximal portion of the inner shield extends when the needle cover is covering the needle.
Clause 8: The needle cover of Clause 7, wherein, as the outer shield is pulled or pushed in a distal direction, the proximal portion of the outer shield contacts the proximal portion of the inner shield held in the at least one opening of the outer shield to cause the inner shield to also move in the distal direction.
Clause 9: The needle cover of Clause 8, wherein, as the inner shield is moved in the distal direction, the extension strip of the outer shield interleaves between the tip and the inner shield to peel or separate the inner shield from the tip.
Clause 10: The needle cover of Clause 7, wherein a longitudinal length of the at least one opening is the same as or substantially similar to a longitudinal length of the extension strip of the outer shield.
Clause 11: A medical injection device, comprising: a barrel extending a proximal face to a distal face, wherein a tip is provided on the distal face of the barrel; and a needle cover for protecting a needle mounted on the tip of the medical injection device, the needle cover, comprising: an inner shield extending along a longitudinal axis, and an outer shield surrounding at least partially the inner shield, and operatively connected to said inner shield, the medical injection device being characterized in that: the outer shield further comprises an extension strip provided on a proximal portion of the outer shield and extends in a distal direction to a position held between the inner shield and the tip.
Clause 12: The medical injection device of Clause 11, wherein the extension strip contacts an inner surface of the inner shield when the outer shield of the needle cover is covering the needle.
Clause 13: The medical injection device of Clause 11, wherein the extension strip does not contact the tip or the inner shield when the outer shield of the needle cover is covering the needle.
Clause 14: The medical injection device of Clause 11, wherein the extension strip is made of a same material as the outer shield.
Clause 15: The medical injection device of Clause 11, wherein the extension strip is made of a rigid plastic material.
Clause 16: The medical injection device of Clause 11, wherein the extension strip is formed integral with the outer shield.
Clause 17: The medical injection device of Clause 11, wherein the outer shield defines at least one opening through which a proximal portion of the inner shield extends when the needle cover is covering the needle.
Clause 18: The medical injection device of Clause 17, wherein, as the outer shield is pulled or pushed in a distal direction, the proximal portion of the outer shield contacts the proximal portion of the inner shield held in the at least one opening of the outer shield to cause the inner shield to also move in the distal direction.
Clause 19: The medical injection device of Clause 18, wherein, as the inner shield is moved in the distal direction, the extension strip of the outer shield interleaves between the tip and the inner shield to peel or separate the inner shield from the tip.
Clause 20: The medical injection device of Clause 17, wherein a longitudinal length of the at least one opening is the same as or substantially similar to a longitudinal length of the extension strip of the outer shield.
Clause 21: The medical injection device of any of Clauses 11-20, wherein the inner shield is made of a soft material including an elastomer or a thermoplastic elastomer.

### BRIEF DESCRIPTION OF THE DRAWINGS

FIG. 1 is a perspective view of a medical injection device according to one example of the present disclosure;
FIG. 2 is a cross-sectional view of the medical injection device of FIG. 1;
FIG. 3 is another cross-sectional view of the medical injection device of FIG. 1;
FIG. 4 is a cross-sectional view of the medical injection device according to another example of the present disclosure;
FIG. 5 is an enlarged view of the medical injection device of FIG. 4;
FIG. 6 is a cross-sectional view of the medical injection device of FIG. 4 in which an inner shield is held on a tip of the medical injection device;
FIG. 7 is a cross-sectional view of the medical injection device of FIG. 4 in which the inner shield is removed from the tip of the medical injection device;
FIG. 8 is a cross-sectional view of the medical injection device according to another example of the present disclosure;
FIG. 9 is an enlarged view of the medical injection device of FIG. 4;
FIG. 10 is a cross-sectional view of the medical injection device of FIG. 4 in which an inner shield is held on a tip of the medical injection device;
FIG. 11 is a cross-sectional view of the medical injection device of FIG. 4 in which the inner shield is at an intermediate position but not fully removed from the tip of the medical injection device; and
FIG. 12 is a cross-sectional view of the medical injection device of FIG. 4 in which the inner shield is removed from the tip of the medical injection device.

### DESCRIPTION OF THE DISCLOSURE

The following description is provided to enable those skilled in the art to make and use the described embodiments contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary embodiments of the invention. Hence, specific dimensions and other physical characteristics related to the embodiments disclosed herein are not to be considered as limiting.

In the following discussion, "distal" refers to a direction generally toward an end of a medical injection device adapted for contact with a patient's skin, and "proximal" refers to the opposite direction of distal, i.e., away from the end of a medical injection device. In other words, the "distal direction" is to be understood as meaning the direction of injection. The distal direction corresponds to the travel direction of the plunger during the injection, the medical composition contained initially in the barrel being expelled from the latter. The "proximal direction" is to be understood as meaning the opposite direction to said direction of injection. For purposes of this disclosure, the above-mentioned references are used in the description of the components of a medical injection device in accordance with the present disclosure.

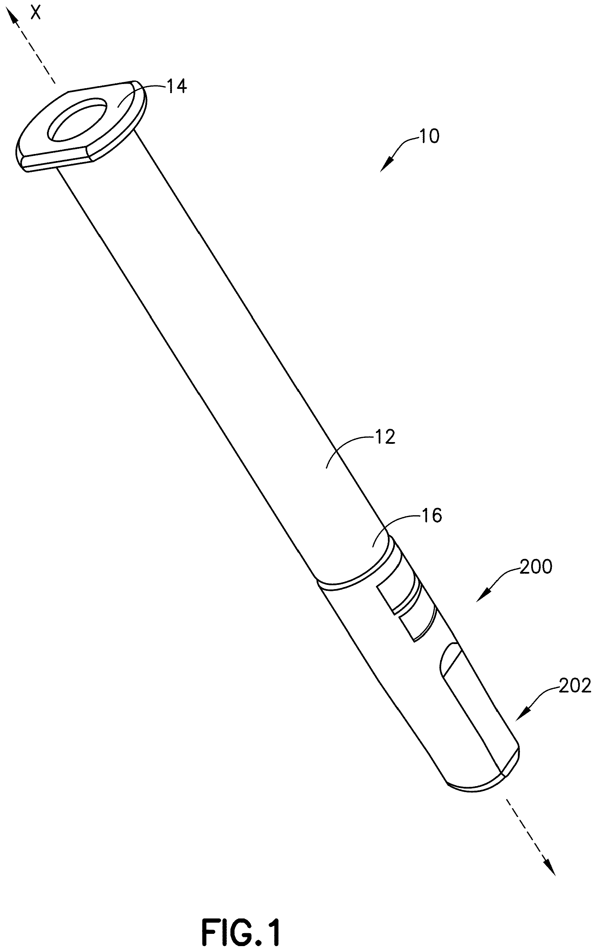

Referring to FIGS. 1-3, the medical injection device 10 may include a barrel 12 extending along a longitudinal axis X from a proximal face 14 to a distal face 16, defining a reservoir 18 for a medical composition such as a liquid medicament. The medical injection device 10 may also include a stopper (not shown), and a plunger rod (not shown) translationally movable inside the barrel 12 from a proximal position to a distal position for injecting the composition.

The medical injection device 10 further includes a distal tip 20 extending along the longitudinal axis X from the distal face 16 of the barrel 12. The distal tip 20 may be at least partially hollow so as to form a fluid path in fluidic communication with the barrel 12. A needle 22 may be attached to the distal tip 20 of the medical injection device 10 and may be in fluid communication with the fluid path. It is noted herein that the distal face 16 of the barrel 12 is proximate a shoulder of the medical injection device 10.

The medical injection device 10 is preferably made of glass, and more preferably is a glass syringe. Such glass syringes are largely used in hospital environments and readily sterilizable. In other examples of the present disclosure, the medical injection device 10 may be made of a medical grade plastic. The medical injection device 10 is preferably a prefilled or a pre-fillable syringe. The medical injection device 10 is more preferably a syringe with the needle 22.

With continued reference to FIGS. 1-3, the medical injection device 10 may further include a needle cover 200. The needle cover 200 may shield and cover the needle 22. The needle cover 200 may include an outer shield 202 and an inner shield 204 that covers the needle 22. In one embedment, the outer shield 202 covers at least a portion of the inner shield 204. In another embodiment, the outer shield 202 covers the entire inner shield 204.

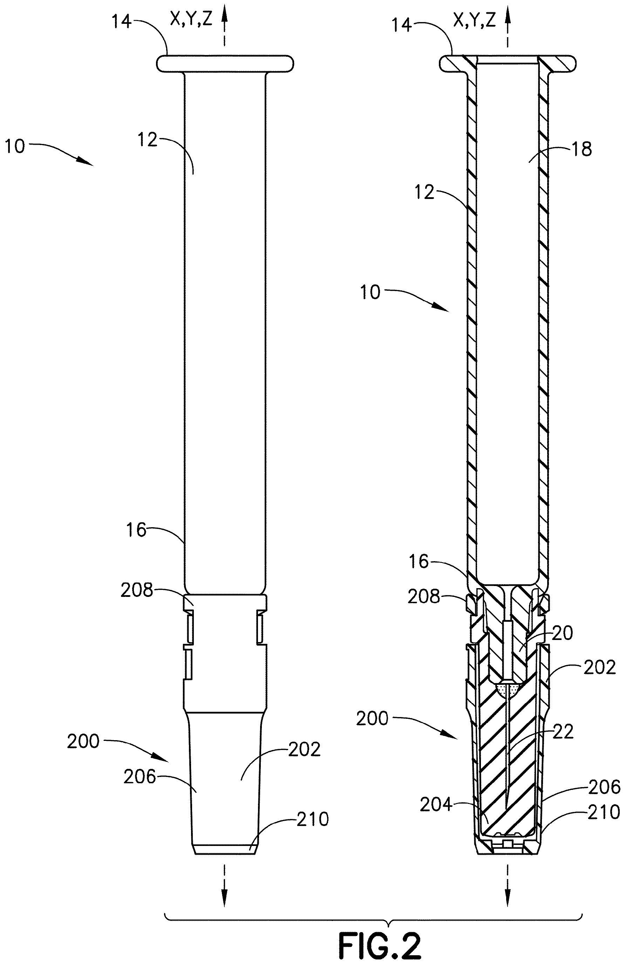

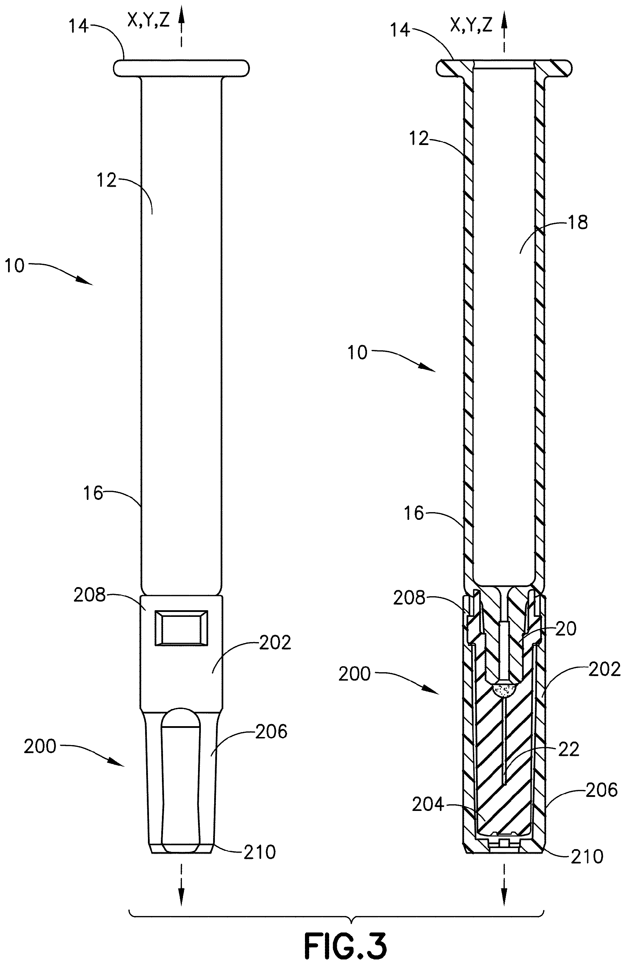

Referring to FIGS. 2 and 3, the outer shield 202 in accordance with an example of the present disclosure is described in greater detail. The outer shield 202 is preferably made in a rigid material, such as rigid plastic. The outer shield 202 may be made of a medical grade plastic. The rigid material confers rigidity to the outer shield 202, which allows said outer shield 202 to better protect the needle cover 200 from shocks. The structural integrity of the needle cover 200 is thereby improved.

The outer shield 202 includes a body 206 having a proximal end 208 and a distal end 210. The body 206 extends along a longitudinal axis Y. The longitudinal axis Y coincides with the longitudinal axis X when the needle cover 200 is mounted on the tip 20 of the medical injection device 10.

In one example, the body 206 may be substantially cylindrical in shape. The body 206 may define an inner cavity 212 that at least partially receives the inner shield 204 of the needle cover 200. In one embodiment, the distal end 210 of the outer shield 202 may include at least one opening for providing sterilization to the needle cover 200.

With continued reference to FIGS. 2 and 3, the inner shield 204 according to one example of the present disclosure is described. The inner shield 204 may extend along a longitudinal axis Z. The longitudinal axis Z may coincide with the longitudinal axis Y when the inner shield 204 is at least partly enclosed in the outer shield 202. The inner shield 204 preferably has a cylindrical shape and a circular cross section.

The inner shield 204 is preferably made in a material with elastomeric properties. The inner shield 204 may be made of a soft material including an elastomer or a thermoplastic elastomer. In this way, at least a portion of the inner shield 204 may slightly deform when connecting the inner shield 204 to the medical injection device 10 so as to match the shape of the tip 20. Meanwhile, the needle tip 20, or distal part of the needle 22, may penetrate the inner shield 204. This further reduces the risk of leakage of the medical composition via the needle 22 to the external environment. The material with elastomeric properties is preferably a thermoplastic elastomer, an elastomer, or a rubber. Preferably, the material with elastomeric properties is sterilizable.

In one embodiment, the outer shield 202 surrounds at least partially the inner shield 204. The outer shield 202 is fixed to the inner shield 204. The inner shield 204 and the outer shield 202 may be fixed together by a snap-fit connection or a friction fit, among other connection methods. When the needle cover 200 is mounted on the medical injection device 10, the inner shield 204 encloses at least a portion of the tip 20 of the barrel 12. The needle cover 200 is thus tightly and sealingly connected to the tip 20. In one embodiment, the inner shield 204 creates a sufficiently tight seal with the tip 20 of the medical injection device 10 to ensure the sterility and fluid tight seal between the tip 20 of the medical injection device 10 and the inner shield 204.

In order to mount the needle cover 200 on the medical injection device 10, the outer shield 202 is first placed over the inner shield 204, and then the entire needle cover 200 is mounted on the medical injection device 10. After the needle cover 200 has been positioned on the medical injection device 10, the needle cover 200 can be moved in a distal direction to remove it from the medical injection device 10. As a pulling force is applied to the outer shield 202 by a user, the outer shield 202 is moved in the distal direction along with the inner shield 204. As the outer shield 202 is moved in the distal direction, a proximal end 208 of outer shield 202 will begin to contact and abut a proximal portion 220 of the inner shield 204. Once the proximal end 208 of the outer shield 202 contacts the proximal portion 220 of the inner shield 204, the proximal end 208 begins to push the proximal portion 220 in a distal direction. As the proximal portion 220 is pushed in the distal direction, the inner shield 204 is pulled in a radial direction away from the medical injection device 10.

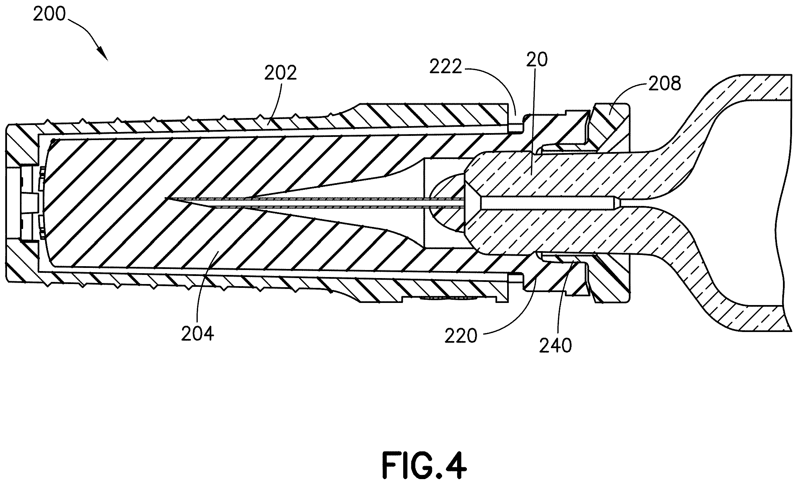

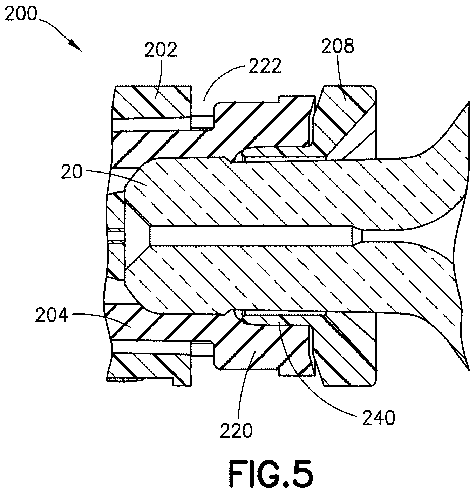

With reference to FIGS. 4 and 5, the inner shield 204 includes the proximal portion 220 that receives the tip 20 of the medical injection device 10. The inner shield 204 creates a seal with the tip 20 of the medical injection device 10. The proximal portion 220 of the inner shield 204 extends radially outward through openings 222 defined in the outer shield 202. Therefore, as the outer shield 202 is pulled or pushed in a distal direction by a user, the proximal end 208 of the outer shield 202 abuts the proximal portion 220 of the inner shield 204 and forces the inner shield 204 to also move in the distal direction.

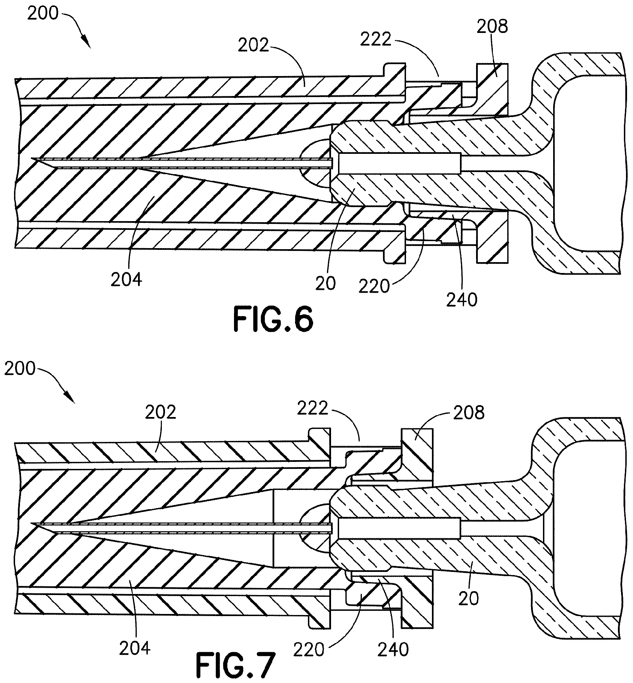

According to one non-limiting embodiment or aspect of the present disclosure, the outer shield 202 of the needle shield 200 may also include an extension strip 240 that is provided on or adjacent to the proximal end 208 of the outer shield 202. The extension strip 240 may be formed of the same material as the outer shield 202, such as a rigid plastic material. The extension strip 240 may be formed integral with the outer shield 202. The extension strip 240 extends in a distal direction from the proximal end 208 of the outer shield 202 to a position at which the extension strip 240 rests between the tip 20 of the medical injection device 10 and the proximal portion 220 of the inner shield 204. In one example, the extension strip 240 makes contact with an inner surface of the proximal portion 220 of the inner shield 204 but does not contact the tip 20 of the medical injection device 200. The extension strip 240 is provided to avoid a non-functional contact area between the rubber or elastomeric inner shield 204 and the glass tip 20 of the medical injection device 10. Instead, the extension strip 240 creates a contact area between the rigid outer shield 202 and glass tip 20 of the medical injection device 10. By creating a contact area between the rigid plastic material of the extension strip 240 of the outer shield 202 and the glass tip 20 of the medical injection device 10 instead of creating a contact area between the rubber or elastomeric material of the inner shield 204 and the glass tip 20 of the medical injection device 10, the pull out force needed to remove the inner shield 204 from the tip 20 of the medical injection device 10 is reduced by deforming the inner shield 204 during removal from the tip 20 of the medical injection device 10. FIGS. 6 and 7 depict the position in which the inner shield 204 is held on the tip 20 of the medical injection device 20 and the position in which the inner shield 204 has been removed from the tip 20 of the medical injection device 20, respectively. In one non-limiting embodiment or aspect of the present disclosure, the longitudinal length of the extension strip 240 is equal to or substantially equal to the longitudinal length of the openings 222 defined in the outer shield 202.

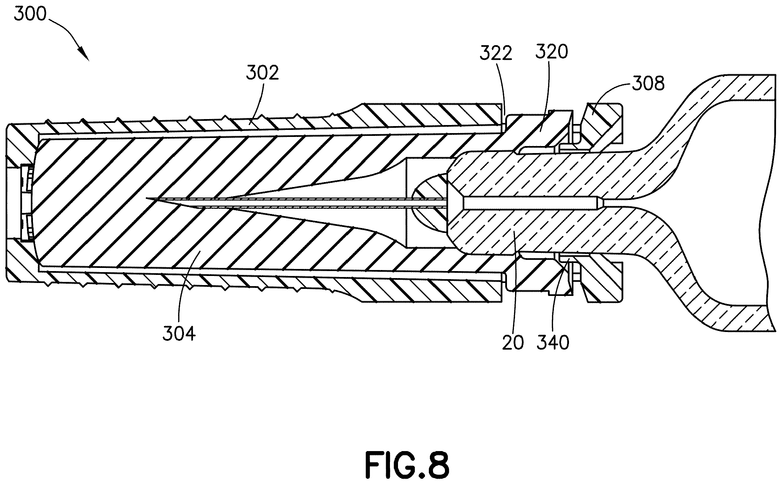

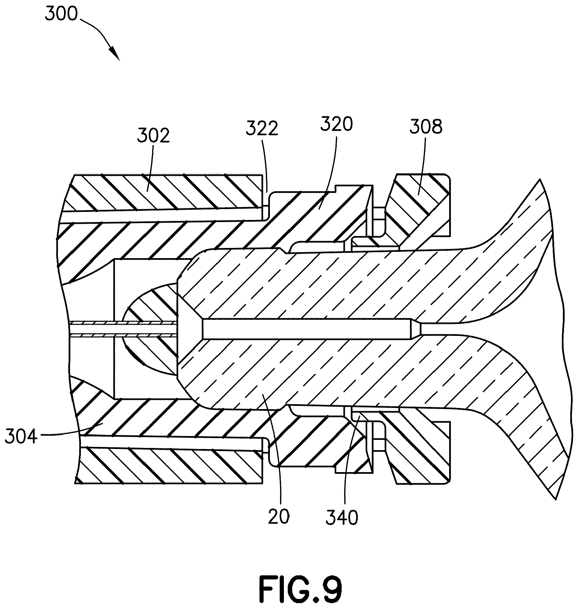

With reference to FIGS. 8 and 9, according to another non-limiting embodiment or aspect of the present disclosure, a needle cover 300 is shown and illustrated. The needle cover 300 is assembled and operates in a similar manner to the needle cover 200 described above but with a few differences with the extension strip 340 of the outer shield 302. In this non-limiting embodiment or aspect, the inner shield 304 includes a proximal portion 320 that receives the tip 20 of the medical injection device 10. The inner shield 304 creates a seal with the tip 20 of the medical injection device 10. The proximal portion 320 of the inner shield 304 extends radially outward through openings 322 defined in the outer shield 302. Therefore, as the outer shield 302 is pulled or pushed in a distal direction by a user, the proximal end 308 of the outer shield 302 abuts the proximal portion 320 of the inner shield 304 and forces the inner shield 304 to also move in the distal direction.

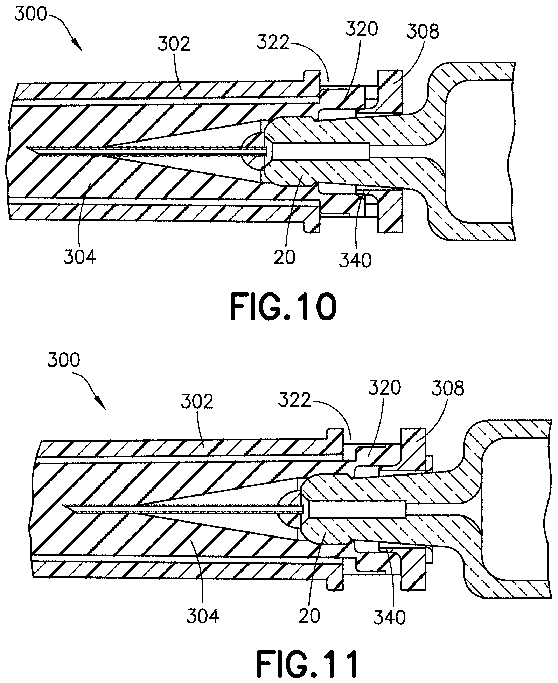

According to one non-limiting embodiment or aspect of the present disclosure, the outer shield 302 of the needle cover 300 may also include an extension strip 340 that is provided on or adjacent to the proximal end 308 of the outer shield 302. The extension strip 340 may be formed of the same material as the outer shield 302, such as a rigid plastic material. The extension strip 340 may be formed integral with the outer shield 302. The extension strip 340 extends in a distal direction from the proximal end 308 of the outer shield 302 to a position at which the extension strip 340 rests between the tip 20 of the medical injection device 10 and the proximal portion 320 of the inner shield 304. In the present example, the extension strip 240 does not make contact with an inner surface of the proximal portion 320 of the inner shield 304 or the tip 20 of the medical injection device 10 when the outer shield 302 and the inner shield 304 are held at a cover position on the tip 20 (see FIG. 10). The extension strip 340 is provided to avoid a non-functional contact area between the rubber or elastomeric inner shield 304 and the glass tip 20 of the medical injection device 10 as the inner shield 304 is being pulled or pushed in a distal direction by the outer shield 302. Instead, the extension strip 340 creates a contact area between the rigid outer shield 302 and glass tip 20 of the medical injection device 10. By creating a contact area between the rigid plastic material of the extension strip 340 of the outer shield 302 and the glass tip 20 of the medical injection device 10, the pull out force needed to remove the inner shield 304 from the tip 20 of the medical injection device 10 is reduced by deforming the inner shield 304 during removal from the tip 20 of the medical injection device 10. The extension strip 340 may interleave between the inner shield 304 and the tip 20 when the outer shield 302 is pulled or pushed from the tip 20 of the medical injection device 10 to peel or separate the inner shield 304 from the tip 20 of the medical injection device 10.

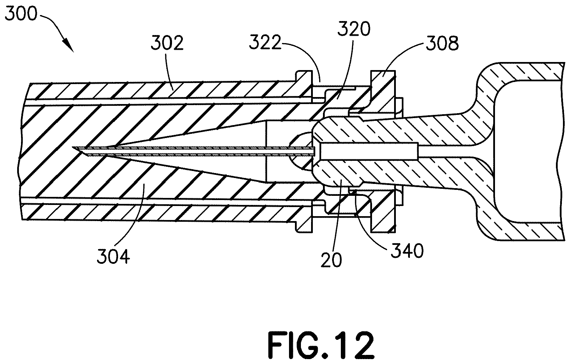

FIGS. 10-12 depict the position in which the inner shield 304 is held on the tip 20 of the medical injection device 10, the intermediate positon in which the extension strip 340 removes a contact area between the inner shield 304 and the tip 20 of the medical injection device 10, and the position in which the inner shield 304 has been removed from the tip 20 of the medical injection device 10, respectively. In the present example of the present disclosure, the extension strip 340 does not eliminate the contact area between the inner shield 304 and the tip 20 of the medical injection device 10 when the inner shield 304 is covering the needle, but, instead, eliminates the contact area between the inner shield 304 and the tip 20 of the medical injection device 10 when the inner shield 304 is being removed from the tip 20 by the outer shield 302.

While this disclosure has been described as having exemplary designs, the present disclosure can be further modified within the spirit and scope of this disclosure. This application is therefore intended to cover any variations, uses, or adaptations of the disclosure using its general principles. Further, this application is intended to cover such departures from the present disclosure as come within known or customary practice in the art to which this disclosure pertains and which fall within the limits of the appended claims.
Further embodiments or aspects are provided in the following numbered clauses:
Clause 1. A needle cover for protecting a tip and a needle mounted on the tip of a medical injection device, wherein the tip extends from a distal end of the medical injection device, the needle cover, comprising:
   an inner shield extending along a longitudinal axis, and
   an outer shield surrounding at least partially the inner shield, and operatively connected to said inner shield,
   the needle cover being characterized in that:
      the outer shield further comprises an extension strip provided on a proximal portion of the outer shield and extends in a distal direction to a position held between the inner shield and the tip.
Clause 2. The needle cover of clause 1, wherein the extension strip contacts an inner surface of the inner shield when the outer shield of the needle cover is covering the needle.
Clause 3. The needle cover of clause 1, wherein the extension strip does not contact the tip or the inner shield when the outer shield of the needle cover is covering the needle.
Clause 4. The needle cover of any of clauses 1-3, wherein the extension strip is made of a same material as the outer shield.
Clause 5. The needle cover of any of clauses 1-4, wherein the extension strip is made of a rigid plastic material.
Clause 6. The needle cover of any of clauses 1-5, wherein the extension strip is formed integral with the outer shield.
Clause 7. The needle cover of any of clauses 1-6, wherein the outer shield defines at least one opening through which a proximal portion of the inner shield extends when the needle cover is covering the needle.
Clause 8. The needle cover of clause 7, wherein, as the outer shield is pulled or pushed in a distal direction, the proximal portion of the outer shield contacts the proximal portion of the inner shield held in the at least one opening of the outer shield to cause the inner shield to also move in the distal direction.
Clause 9. The needle cover of clause 8, wherein, as the inner shield is moved in the distal direction, the extension strip of the outer shield interleaves between the tip and the inner shield to peel or separate the inner shield from the tip.
Clause 10. The needle cover of clause 7, wherein a longitudinal length of the at least one opening is the same as or substantially similar to a longitudinal length of the extension strip of the outer shield.
Clause 11. A medical injection device, comprising:
   a barrel extending a proximal face to a distal face, wherein a tip is provided on the distal face of the barrel; and
   a needle cover for protecting a needle mounted on the tip of the medical injection device, the needle cover, comprising:
      an inner shield extending along a longitudinal axis, and
      an outer shield surrounding at least partially the inner shield, and operatively connected to said inner shield,
   the medical injection device being characterized in that:
      the outer shield further comprises an extension strip provided on a proximal portion of the outer shield and extends in a distal direction to a position held between the inner shield and the tip.
Clause 12. The medical injection device of clause 11, wherein the extension strip contacts an inner surface of the inner shield when the outer shield of the needle cover is covering the needle.
Clause 13. The medical injection device of clause 11, wherein the extension strip does not contact the tip or the inner shield when the outer shield of the needle cover is covering the needle.
Clause 14. The medical injection device of any of clauses 11-13, wherein the extension strip is made of a same material as the outer shield.
Clause 15. The medical injection device of any of clauses 11-14, wherein the extension strip is made of a rigid plastic material.
Clause 16. The medical injection device of any of clauses 11-15, wherein the extension strip is formed integral with the outer shield.
Clause 17. The medical injection device of any of clauses 11-16, wherein the outer shield defines at least one opening through which a proximal portion of the inner shield extends when the needle cover is covering the needle.
Clause 18. The medical injection device of clause 17, wherein, as the outer shield is pulled or pushed in a distal direction, the proximal portion of the outer shield contacts the proximal portion of the inner shield held in the at least one opening of the outer shield to cause the inner shield to also move in the distal direction.
Clause 19. The medical injection device of clause 18, wherein, as the inner shield is moved in the distal direction, the extension strip of the outer shield interleaves between the tip and the inner shield to peel or separate the inner shield from the tip.
Clause 20. The medical injection device of clause 17, wherein a longitudinal length of the at least one opening is the same as or substantially similar to a longitudinal length of the extension strip of the outer shield.
Clause 21. The medical injection device of any of clauses 11-20, wherein the inner shield is made of a soft material including an elastomer or a thermoplastic elastomer.

## Claims

1. A needle cover for protecting a tip and a needle mounted on the tip of a medical injection device, wherein the tip extends from a distal end of the medical injection device, the needle cover, comprising:
an inner shield extending along a longitudinal axis, and
an outer shield surrounding at least partially the inner shield, and operatively connected to said inner shield,
the needle cover being **characterized in that**:
the outer shield further comprises an extension strip provided on a proximal portion of the outer shield and extends in a distal direction to a position held between the inner shield and the tip.

2. The needle cover of claim 1, wherein the extension strip contacts an inner surface of the inner shield when the outer shield of the needle cover is covering the needle.

3. The needle cover of claim 1, wherein the extension strip does not contact the tip or the inner shield when the outer shield of the needle cover is covering the needle.

4. The needle cover of any of claims 1-3, wherein the extension strip is made of a same material as the outer shield.

5. The needle cover of any of claims 1-4, wherein the extension strip is made of a rigid plastic material.

6. The needle cover of any of claims 1-5, wherein the extension strip is formed integral with the outer shield.

7. The needle cover of any of claims 1-6, wherein the outer shield defines at least one opening through which a proximal portion of the inner shield extends when the needle cover is covering the needle.

8. The needle cover of claim 7, wherein, as the outer shield is pulled or pushed in a distal direction, the proximal portion of the outer shield contacts the proximal portion of the inner shield held in the at least one opening of the outer shield to cause the inner shield to also move in the distal direction.

9. The needle cover of claim 8, wherein, as the inner shield is moved in the distal direction, the extension strip of the outer shield interleaves between the tip and the inner shield to peel or separate the inner shield from the tip.

10. The needle cover of claim 7, wherein a longitudinal length of the at least one opening is the same as or substantially similar to a longitudinal length of the extension strip of the outer shield.

11. A medical injection device, comprising:
a barrel extending a proximal face to a distal face, wherein a tip is provided on the distal face of the barrel; and
a needle cover for protecting a needle mounted on the tip of the medical injection device, as claimed in claims 1 to 10.

12. The medical injection device of claim 11, wherein the inner shield is made of a soft material including an elastomer or a thermoplastic elastomer.
